# EUROPEAN PATENT SPECIFICATION

(11) **EP 3 536 281 B1**
(45) Date of publication and mention of the grant of the patent: **20.04.2022**
(21) Application number: 19171119.1
(22) Date of filing: 19.09.2013
(51) Int. Cl.: A61F 2/16

(54) **INTRAOCULAR LENS**
INTRAOKULARLINSE
LENTILLE INTRAOCULAIRE

(30) Priority: 19.09.2012 US 201261702995 P
(43) Date of publication of application: 11.09.2019
(62) Divisional of application: 13839938.1
(73) Proprietor: Zaldivar, Roger, Miami, FL 33154 (US); Zaldivar, Roberto, Mendoza (AR)
(72) Inventor: Zaldivar, Roger, Miami, FL 33154 (US); Zaldivar, Roberto, Mendoza (AR)
(74) Representative: Elzaburu S.L.P.

(56) References cited:
- WO-A1-2012/092333
- US-A- 3 558 369
- US-A- 4 731 079

## Description

### Field of the invention

This disclosure relates to intraocular lenses for the improvement and correction of vision.

### Background of the invention

The human eye is susceptible to a number of problems that affect the quality of vision. For example, as the eye ages, there is a loss of the ability to focus on near objects, a disorder known as presbyopia. Presbyopia is typically linked to a loss of elasticity of the crystalline lens. Another age-related problem is cataract formation, i.e., a clouding of the crystalline lens. This causes scattering, diminishment or complete blockage of optical light.

Vision quality is also affected by nearsightedness- known as myopia-in which the crystalline lens focuses light in front of the retina instead of directly on the retina. Figure 1 illustrates a normally functioning eye in which lens 4 focuses light on retina 10. In contrast, Figure 2 illustrates a myopic eye wherein lens 4 focuses light at a point 100 in front of the retina 10. Because light is focused in front of the retina 10, far-away objects appear blurry. Likewise, vision quality can be affected by farsightedness-known as hyperopia-in which the crystalline lens focuses light behind the retina, as shown in Figure 3. Farsightedness makes near objects appear blurry.

Yet another frequent eye problem is astigmatism. Astigmatism is an optical defect in which vision is blurred due to the inability of the optics of the eye to focus a point object into a sharp focused image on the retina that may be due to an irregular or toric curvature of the cornea or lens.

### Prior art IOLs

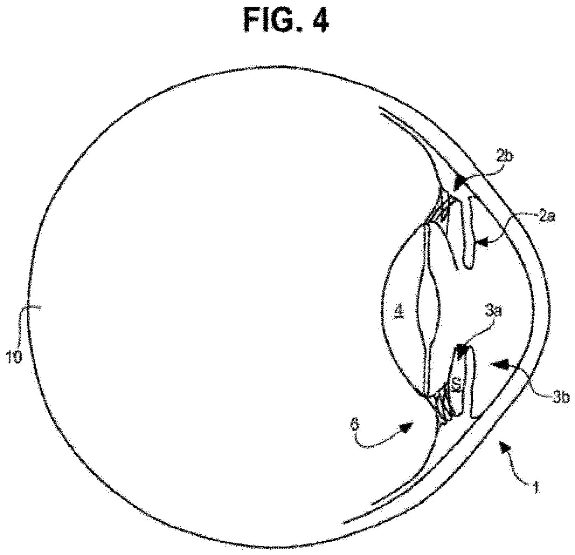

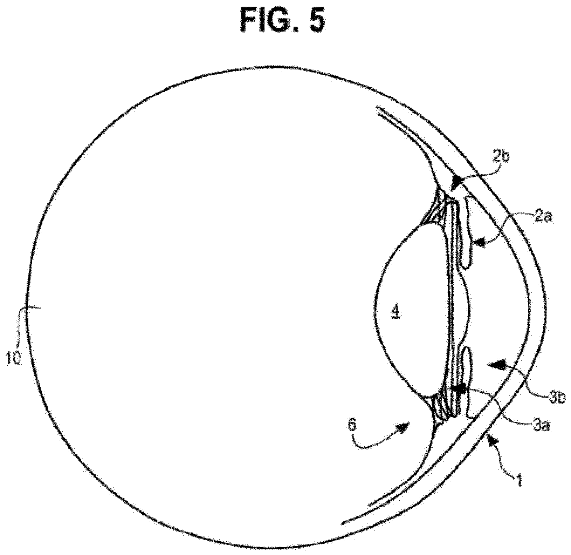

Presbyopia, cataracts, myopia, astigmatism and a number of other eye-related problems can be dramatically improved or altogether corrected by surgical implantation of an intraocular lens (IOL). IOLs can be used to completely replace the natural lens, as shown in Figure 4. Alternatively, a type of IOL, known as a phakic IOL (PIOLs), can be implanted in front of the natural lens (posterior chamber phakic IOLs), or in the anterior chamber (iris fixated phakic IOL and angle supported phakic IOL), thus altering the power of the entire eye. PIOLs are illustrated in Figures 5-6.

Presently available IOLs are formed from a flexible, plastic lens. In addition, each IOL has flexible, radial struts or wings-known as haptics-that secure the lens within the eye. This configuration allows a physician to collapse or roll the IOL into a compact shape for implantation, and then unroll and position the IOL within the eye.

IOL lenses have either a monofocal or multifocal refractive value or diopter. To achieve ideal vision, also known as emmetropia, a physician must properly calculate the appropriate refractive power and size particularly in phakic IOLs suitable for each eye.

US-A-4731079 discloses an intraocular lens including at least a non-toxic, biocompatible, hydrolytically and enzymatically stable, photodegradation resistant, polymeric optical zone portion, wherein said polymeric optical zone portion has the following characteristics, when in osmotic equilibrium with body liquids: a) softening temperature Ts between about 0º C and about 42º C; b) damaging temperature Td higher than 42º C; c) refractive index greater than 1.39; and d) temperature range of elastic deformation Te above Ts but below Td; and when said polymeric optical zone portion is heated to Te one of its original dimensions is reduced by at least 20%, then cooled to at least 5ºC below Tsand, upon reheating to Te said polymeric optical zone portion will return to its original dimensions existing prior to said first heating, dimensional reduction and cooling.

WO 2012092333 A1 discloses an implantable ophthalmic device with one or more optical elements coupled to one or more shape-memory members that provides dynamically variable optical power to restore lost accommodation in individuals suffering from presbyopia or aphakia. Running current from a power supply through the shape-memory members causes the shape-memory members to heat up. Once the current heats the shape-memory members past a forward phase-transition temperature, the shape-memory members change shape, which, in turn, causes the optical element(s) to move, yielding a corresponding change in effective optical power. Cooling the shape-memory members (e.g., by reducing or stopping the flow of current) below a reverse phase-transition temperature causes the shape-memory members to return to their original shape, which, in turn, restoring the optical element(s) to their original positions and returning the effective optical power to its original level.

### Drawbacks of prior art IOLs

While IOLs have drastically improved treatment of many eye problems, IOLs still have significant drawbacks.

One significant drawback to presently available IOLs relates to selection of the ideal diopter for the IOL for a particular eye. In particular, selection of an IOL having an incorrect diopter for a particular eye leads to post-surgical refractive residual error (myopia, hyperopia or astigmatism). In spite of recent improvements in ophthalmic devices and methods for diopter calculation, the resulting selected diopter is not ideal. Indeed, studies of lens replacement patients treated by an experienced ophthalmologist show that about 50% of patients are not entirely satisfied with the surgical outcome and likely need an optical correction to appropriately to further improve their vision.

Another drawback to presently available IOLs relates to misalignment of toric IOLs (astigmatism correction). In cases involving astigmatism, misalignment of the IOL may result in a residual astigmatism.

Another significant drawback to presently available IOLs occurs during cataract surgery. During cataract surgery, the cataract is removed from the sac that surrounds it, also called the capsular bag. The capsular bag remains in place because of the support provided by the zonule. The IOL is implanted most frequently inside the capsular bag in cataract surgery, as shown in Figure 4. In some cases, the IOL is implanted outside the capsular bag, most frequently at the sulcus. The power of the IOL will depend in the final position inside the eye being stronger if it shifts anteriorly or weaker if it shifts posteriorly. This is known as the estimated lens position (ELP) and is the most difficult thing to predict during the calculation of the IOL and the main source of error.

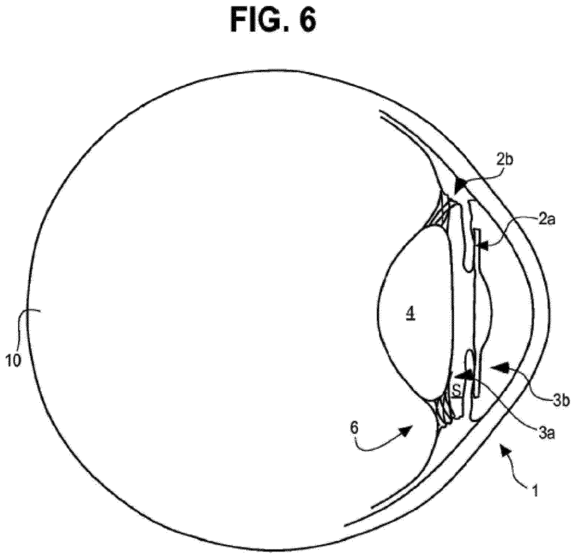

Yet another significant drawback relates to the requirement that a PIOL be positioned precisely relative to the natural lens or the endothelium. PIOLs can be positioned either in the posterior or anterior chamber of any eye. For PIOLs implanted in the posterior chamber, shown in Figure 5, the desired spacing (gauged as a type 2 vault) between the PIOL and the front of the natural lens ranges from about 500 to about 750 microns. As such, there is almost no margin of error. Positioning can be further complicated by the presence of cataracts and pupillary block. Where a posterior chamber PIOL is too close to the natural lens, even to the point of actual contact with the natural lens (vault of 1 and 0, respectively), the patient is prone to developing cataracts or other opacities. On the other hand, positioning a posterior chamber PIOL too far from the natural lens, (i.e., a vault of 3 or 4), can lead to compression of the iridocorneal angle, and in some cases to pupillary block and acute glaucoma. For lenses implanted in the anterior chamber, shown in Figure 6, positioning and sizing of the PIOL is crucial. Decompensation of corneal endothelial cell loss, iris atrophy and cataract formation has been described related to this issue. This is the reason why, despite being optically a great solution for addressing the need to use glasses, the considerable number of complications related to PIOLs hinders its global acceptance.

Follow-up surgery is the only present solution to the previously mentioned drawbacks and complications with current IOLs. Surgical options related with residual refractive errors include laser in situ keratomileusis, photorefractive keratomileusis, limbar relaxing incisions, or complete surgical removal and replacement of the IOL or PIOL. Follow-up surgery, however, is extremely undesirable because it results in increased patient discomfort and recovery time, the possibility of further complications, and much higher costs. The sole non-surgical alternative- which is only available alternative in certain circumstances-is to wear glasses. However, even wearing glasses to solve a post-surgical IOL or PIOL problem is also extremely undesirable. Indeed, the desire to dispense with glasses may often be the very reason that a patient may have undergone the surgical procedure to begin with. Surgical options related to errors in sizing and positioning of phakic IOLs include their replacement, phaco surgery, and corneal transplant.

Accordingly, there is an unfulfilled need for an IOL or PIOL that resolves or improves upon one or more of the drawbacks and complications attributable to present IOLs and PIOLs.

### Summary of the invention

One or more of the preceding drawbacks of currently available IOLs and PIOLs are improved and an advance is made in the art by a novel a phakic, pseudophakic, monofocal, or multifocal IOL. According to the present invention, which is defined by claim 1, a novel IOL is provided with haptics that can be repositioned, resized or otherwise adjusted non-invasively after implantation in a patient.

The intraocular lens implant for the correction of vision includes an optical body with a default refractive power, and a haptic operably connected to the optical body, the haptic comprising at least one shape memory alloy having a segment with a transition temperature substantially higher than the body temperature of a human. The segment may be non-linear at temperatures below the transition temperature and substantially linear at temperatures above the transition temperature. The optical body may include a monofocal region, or a multifocal region. The segment may be linear at temperatures below the transition temperature and non-linear at temperatures above the transition temperature. The intraocular lens implant may further include a second haptic operable connected to the optical body, the second haptic comprising a second shape memory alloy having a second segment with a transition temperature substantially higher than the body temperature of a human. The transition temperature is between 50 degrees Celsius and 500 degrees Celsius.

The intraocular lens implant includes an optical body with a default refractive power, and a haptic operably connected to the optical body, the haptic including an expansion zone and at least one shape memory bar provided in or proximate to the expansion zone, a segment of the memory bar having a transition temperature greater than 50 degrees Celsius, wherein the expansion zone of the haptic is deformable when the at least a portion of the memory bar has reached its transition temperature. The embodiment may further include an additional haptic connected to a side of the optical body opposing the haptic, the additional haptic including an expansion zone and at least one shape memory bar provided in or proximate to the expansion zone, a segment of the memory bar having a transition temperature greater than 50 degrees Celsius, and the expansion zone of the additional haptic is deformable when the at least a portion of the memory bar has reached its transition temperature. The haptic may include a plurality of shape memory bars provided in or proximate to the expansion zone of the haptic, and the additional haptic may include a plurality of shape memory bars provided in or proximate to the expansion zone of the additional haptic. The memory bars in either or both the haptic and the additional haptic may be composed of a shape memory alloy, the shape memory alloy comprising nickel titanium. The expansion zone is crinkled.

A method for non-surgically adjusting the position of an implanted intraocular lens, this method not being part of the invention, may include providing an intraocular lens implant for the correction of vision including an optical body with a default refractive power, a haptic operably connected to the optical body, the haptic comprising at least one shape memory alloy having a segment with a transition temperature substantially higher than the body temperature of a human, implanting the intraocular lens within the eye of a patient, post-surgically dilating the eye, providing an electromagnetic wave delivery means, delivering electromagnetic waves with the deliver means to a predetermined section of the segment until the segment reaches the transition temperature, whereby the haptic is deformed so as to exert a desired biasing force within the eye. The biasing force may secure, reposition, or adjust the intraocular lens implant within the eye. The biasing force may also align the intraocular lens implant to a desired position within the eye. The laser may be an argon laser.

Other characteristics and advantages of the invention will be discernible to the ordinarily skilled artisan when equipped with the teachings of the present disclosure. Certain objects and advantages of the present invention may be realized when the instrumentalities and combinations particularly pointed out in the appended claims are considered.

The foregoing and other objectives, features, and advantages of the invention will be more readily understood upon consideration of the following detailed description of the invention.

### Brief description of the drawings

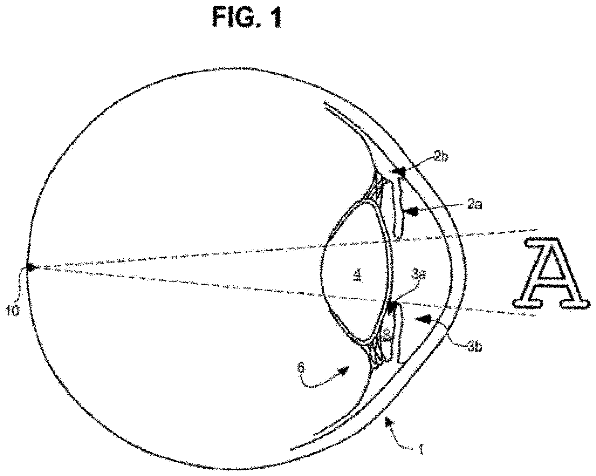

The following numerical descriptors are employed in certain of Figures 1 to 21 to identify the various structural elements being represented: cornea (1); central iris (2a); peripheral iris (2b); posterior chamber (3a); anterior chamber (3b); natural lens (4); sulcus (5); ciliary zonule (6); area of the intraocular lens optic or optical body (107); proximal area of the haptic (108a); distal area of the haptic (108b); retina( 10); memory alloy device shape (11).
Figure 1 is a cross-sectional side view of an eye and its main internal structures, demonstrating the proper focal point of the eye in the visual state of emmetropia.

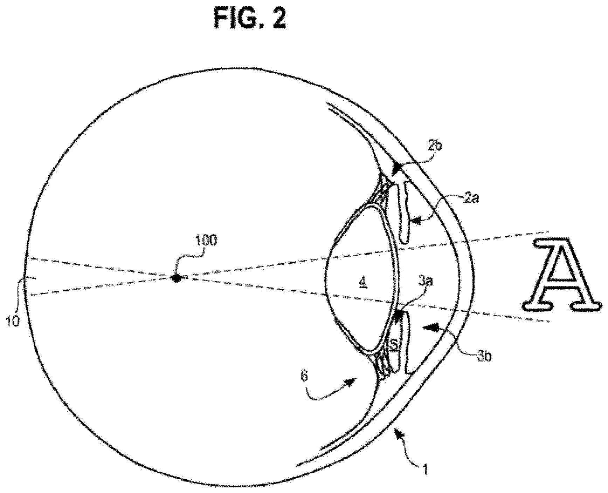

Figure 2 is a cross-sectional side view of an eye and its main internal structures, demonstrating the forward (relative to optimum) focal point of the eye in the visual state of myopia.

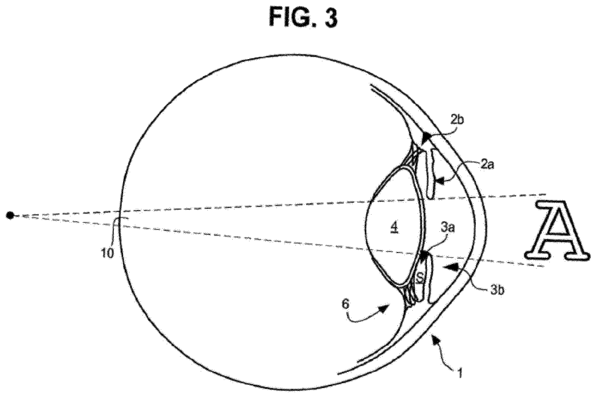

Figure 3 is a cross-sectional side view of an eye and its main internal structures, demonstrating the rearward (relative to optimum) focal point of the eye in the visual state of hyperopia.
Figure 4 is a cross-sectional side view of an eye without a natural lens and having a replacement intraocular lens positioned equatorially inside the capsular bag.
Figure 5 is a cross-sectional side view of an eye with a phakic lens implanted in the posterior chamber.
Figure 6 is a cross-sectional side view of an eye with a phakic lens implanted in the anterior chamber.

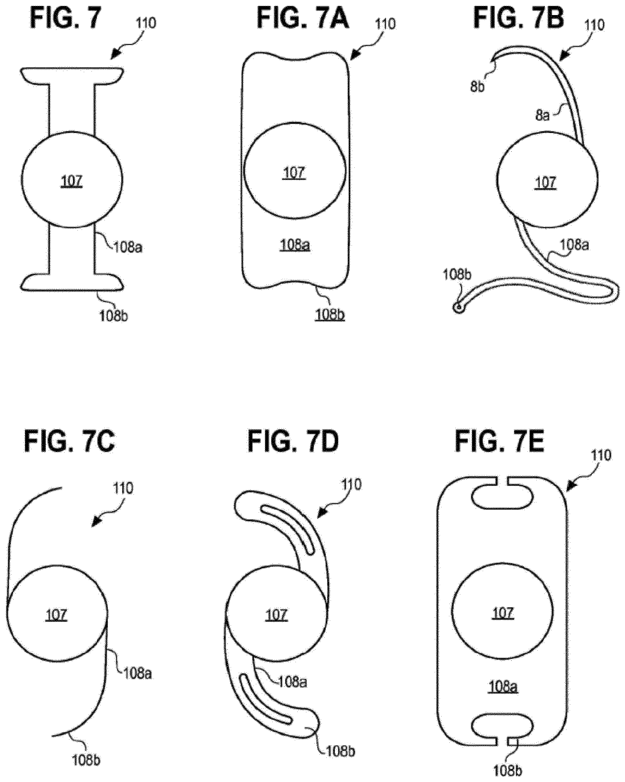

Figure 7 is a collection of front views of different types of intraocular lenses useful in the present invention.

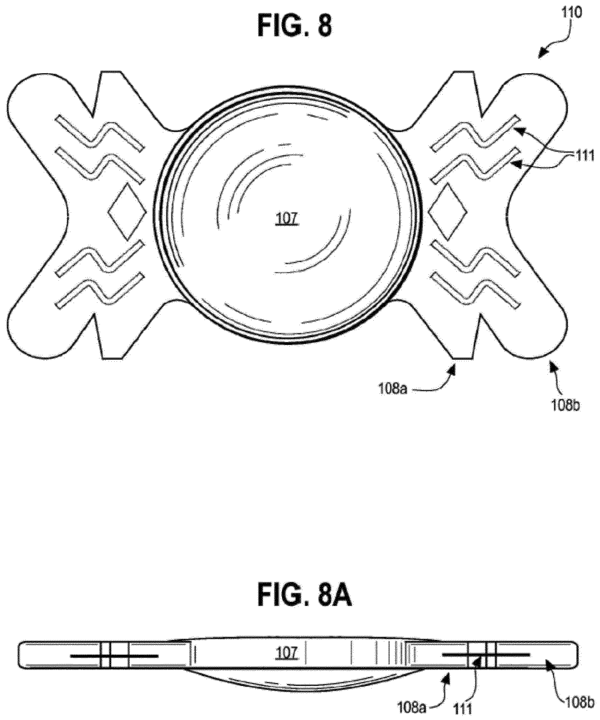

Figure 8 is a front view of an intraocular lens with memory alloy and at least one lens haptic.
Figure 8A is a side view of the intraocular lens of Figure 8.

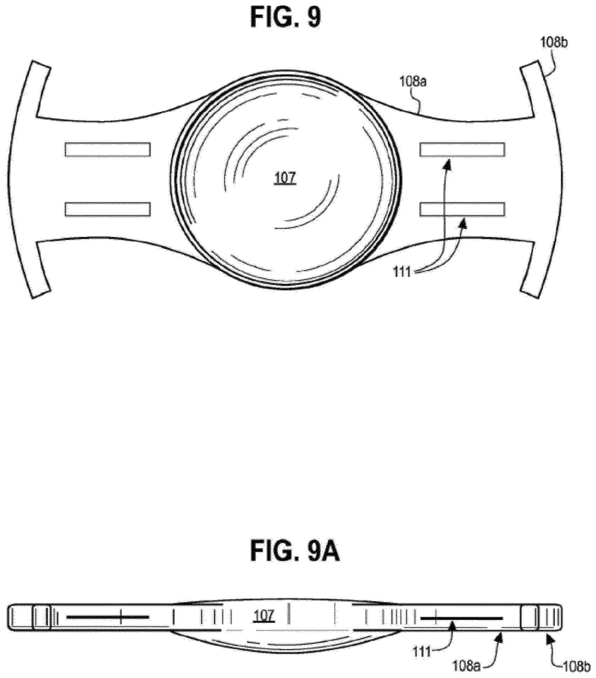

Figure 9 is a front view of an intraocular lens with memory alloy and at least one lens haptic.
Figure 9A is a side view of the intraocular lens of Figure 9.

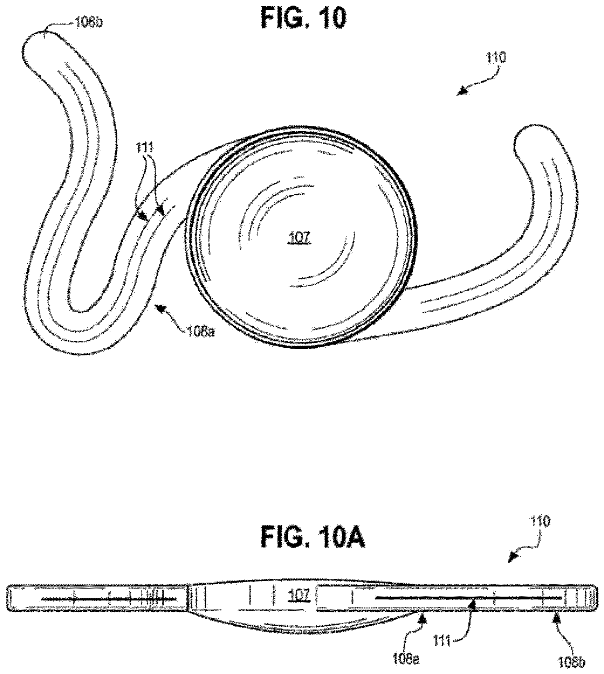

Figure 10 is a front view of an intraocular lens with memory alloy and at least one lens haptic.
Figure 10A is a side view of the intraocular lens of Figure 10.

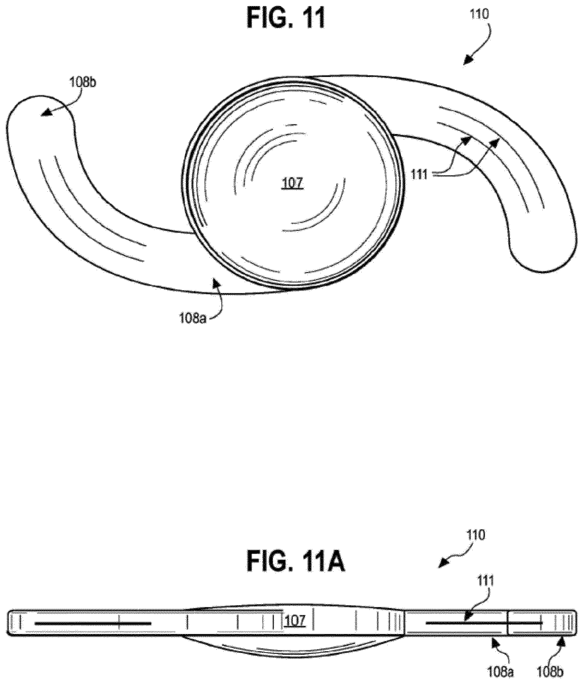

Figure 11 is a front view of an intraocular lens with memory alloy and at least one lens haptic.
Figure 11A is a side view of the intraocular lens of Figure 11.

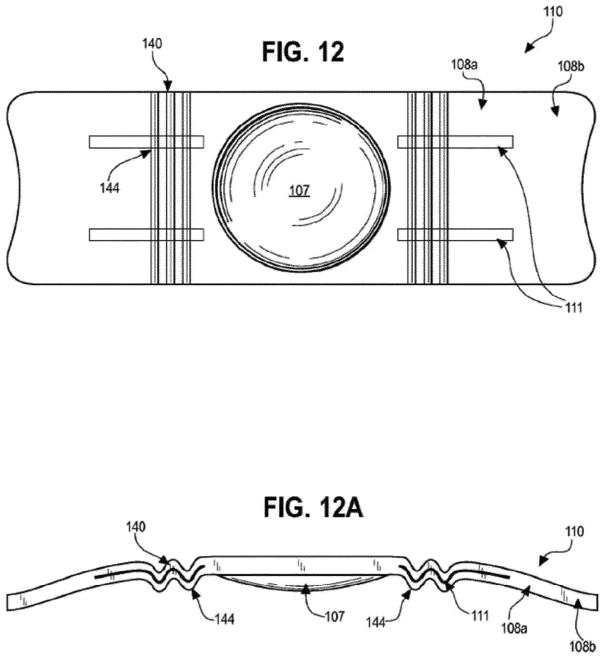

Figure 12 is a front view of an embodiment of an intraocular lens of the invention with memory alloy and at least one lens haptic.
Figure 12A is a side view of the intraocular lens of Figure 12.

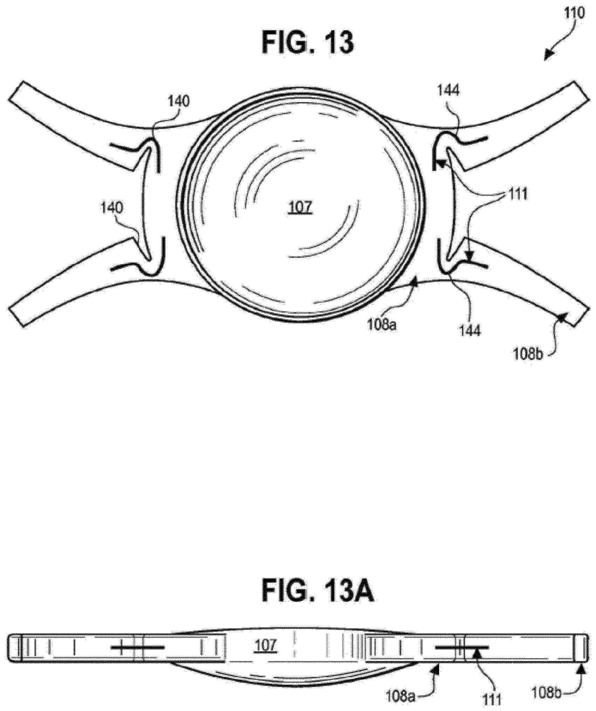

Figure 13 is a front view of an intraocular lens with memory alloy and at least one lens haptic.
Figure 13A is a side view of the intraocular lens of Figure 13.

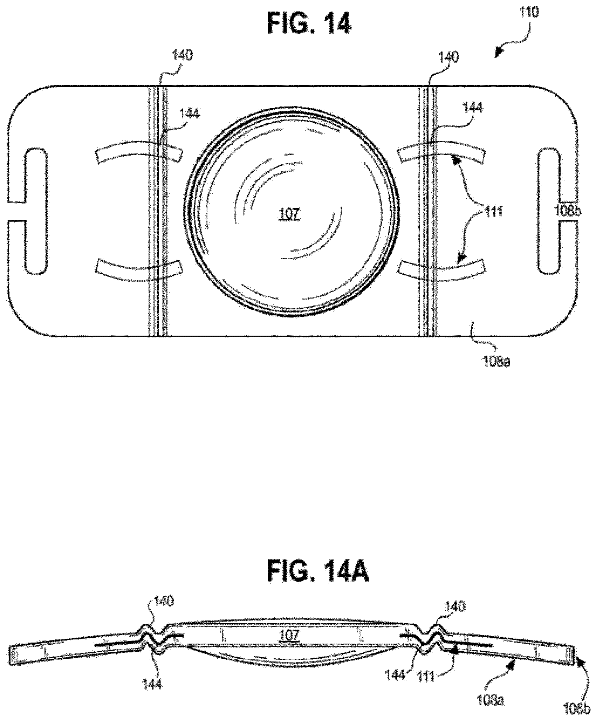

Figure 14 is a front view of an embodiment of an intraocular lens of the invention with memory alloy and at least one lens haptic.
Figure 14A is a side view of the intraocular lens of Figure 14.

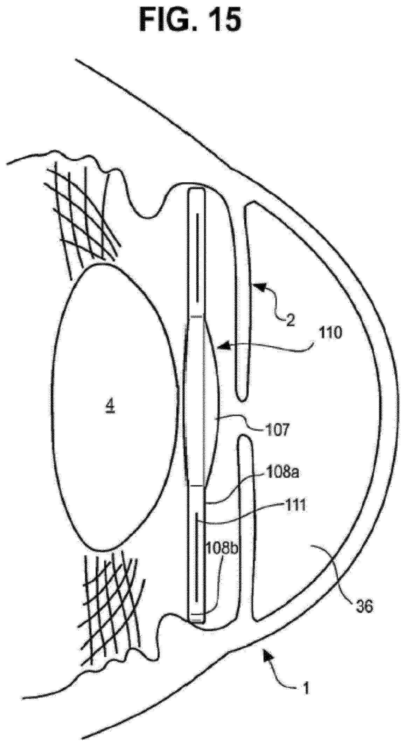

Figure 15 is a cross-sectional schematic side view representation of a posterior- chamber-implanted phakic intraocular lens that is in contact with the natural lens.

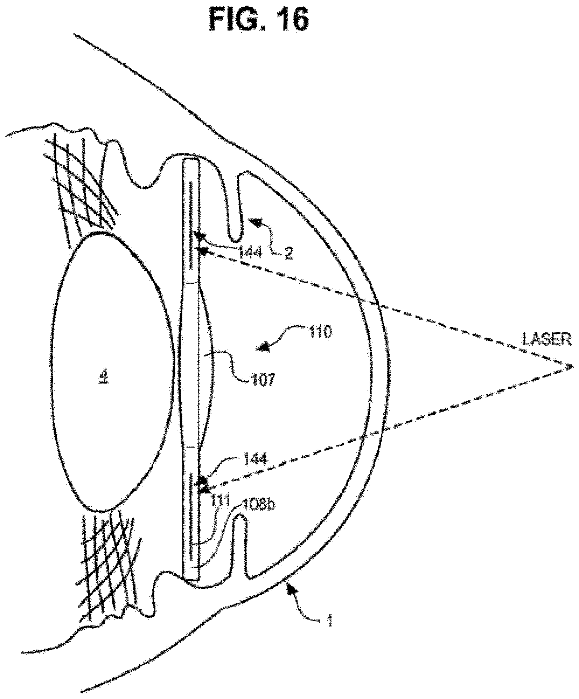

Figure 16 is a cross-sectional side view of a eye with the iris dilated and imaginary axes that may be employed along which the laser beam may be directed to contact the shape memory alloy in a haptic-located device.

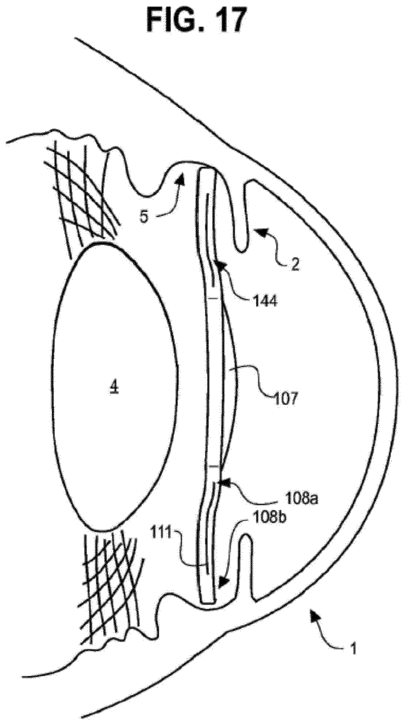

Figure 17 is a cross-sectional side view of a posterior-chamber-implanted phakic intraocular lens having memory shape alloy haptics (containing a memory shape alloy device) bent at an angle that allow repositioning of the implanted lens further away from the natural lens to zoom out the optics of the implanted lens when an appropriate laser beam is applied to the device.

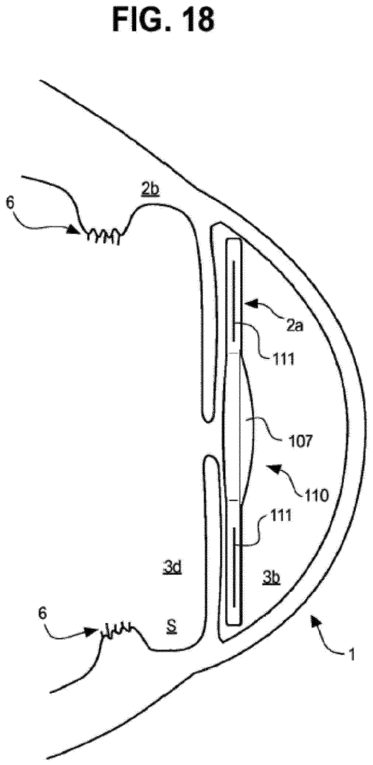

Figure 18 is a cross-sectional side view of an IOL placed in the anterior chamber of the eye.

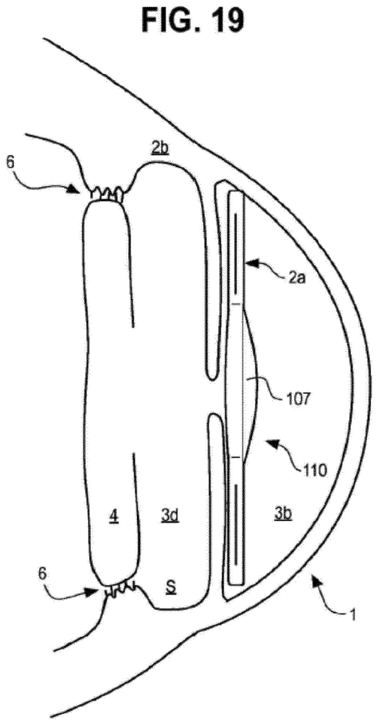

Figure 19 is a cross-sectional side view of an IOL placed in the anterior chamber of the eye and its location with respect to the natural eye's capsular sac left intact after removal of the natural lens.

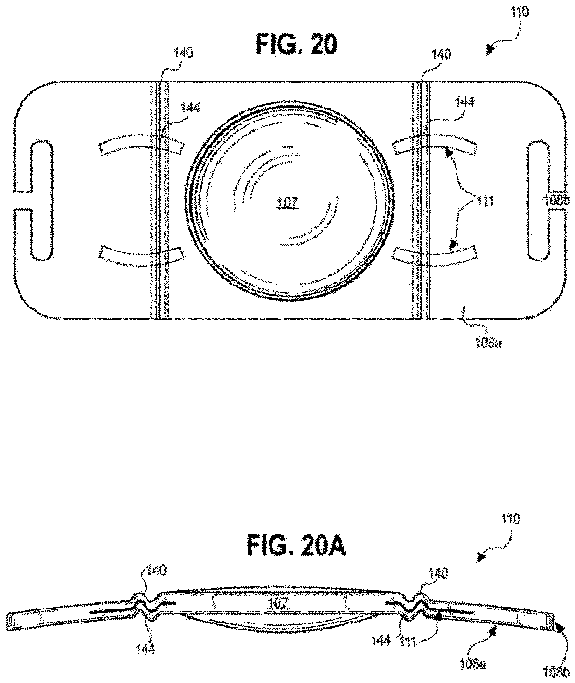

Figure 20 is a front view of an embodiment of an intraocular lens having multiple haptics, each haptic having multiple shape memory alloy devices.
Figure 20A is a side view of the intraocular lens of Figure 20.

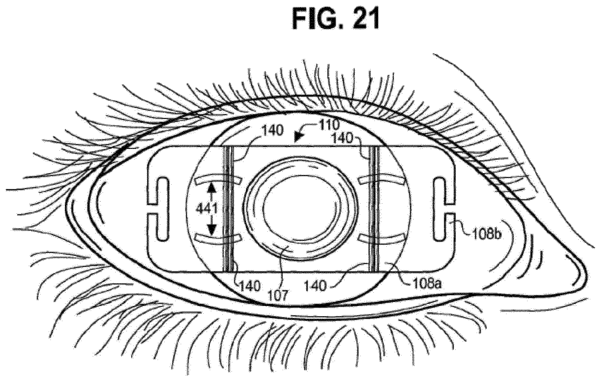

Figure 21 is a front view of an IOL of the present invention positioned in the eye with iris dilated in preparation for adjustments to its positioning with laser irradiation.

### Detailed description of the illustrative embodiments

The following detailed description and the appended drawings describe and illustrate exemplary embodiments of the invention solely for the purpose of enabling one of ordinary skill in the relevant art to make and use the invention. As such, the detailed description and illustration of these embodiments are purely exemplary in nature and are in no way intended to limit the scope of the invention, or its protection, in any manner. It should also be understood that the drawings may not be to scale and in certain instances details have been omitted, which are not necessary for an understanding of the present invention, such as conventional details of fabrication and assembly.

### Definitions

As employed throughout the disclosure of the present invention, the following terms, unless otherwise indicated, shall be understood to have the following meanings.

As used herein, the term "emmetropia" refers to the ideal refractive state of the eye.

As used herein, the term "substantially emmetropic state" refers to refractive states of the eye that reasonably closely approximate the ideal refractive state of the eye, including refractive states influenced by conditions of astigmatism, myopia and hyperopia (e.g., conditions with refractive states diverging from ideal). Typically, refractive states that are substantially emmetropic include states that diverge from the ideal in the range of from about 0 to about 10%. Alternatively, a substantially emmetropic state as used herein would provide vision acuity of at least about 90%, preferably of at least about 95%, more preferably of at least about 98% and still more preferably of at least about 99% of the same eye at emmotropia.

As used herein, the term "anterior chamber" refers to the part of the anterior cavity of the eye in front of the iris that contains the aqueous humor.

As used herein, the term "posterior chamber" refers to that part of the aqueous humor-containing space of the eyeball between the iris and the lens.

As used herein, the term "refractive power" refers to the degree to which a lens, mirror, or other optical system converges or diverges light.

As used herein, the term "shape memory alloy" refers to an alloy that "remembers" its original, cold-forged shape. When a shape-memory alloy is in its cold state, the metal can be bent or stretched and will hold those shapes until heated above the transition temperature. Upon heating, the shape changes to its original forged shape. When the metal cools again it will remain in the hot shape, until deformed again. One exemplary "shape memory alloy" is Nickel Titanium (NiTi), otherwise known as Nitinol.

Referring to the drawings, Figures 7-21 illustrate various embodiments of an IOL 110, each of which generally includes a flexible optical body or lens 107 joined to haptics 108. Figure 7 illustrates a number of embodiments of an IOL having a lens 107 with a default optical or refractive power. In addition, such IOLs have at least one haptic 108 with a proximate area 108a adjacent to the optical body 107, and a distal area 108b at the terminal end of the haptic. Haptic 108 may be formed entirely of a shape memory alloy. Alternatively, haptic 108 may be a flexible plastic material having integral shape memory bars 111.

Figures 7 through 14 illustrate alternative designs of IOL 110 having haptic 108 and shape memory bars 111. As illustrated in Figure 12, shape memory bars 111 are provided with one or more crinkled or bent portions 144 that coincide with the expansion zones 140. Such zones are configured to be heated to elongate, bend more, or even rotate about an axis of the haptic 108. Such zones are preferably identified or marked in such a manner that a physician can select one or more zones to heat in order to achieve a desired change in the position and shape of the haptic.

In one embodiment, shown in Figure 12, IOL 110 is provided in an elongate, rectangular configuration having curvilinear comers, haptics 108, haptic expansion zones 140 and shape memory bar expansion zones 144. Each shape memory bar 111 and expansion zone 140 is configured to remain in a first, closed or retracted position before, during and after the procedure in which IOL 110 is implanted. If adjustment of the position, size, or shape of the IOL 110 is desired, a physician can apply focused, electromagnetic wave on predetermined locations of each shape memory bar 111 until the shape memory bar reaches the a pre-set transition temperature and returns to its forged state. The physician can thus move IOL 110 toward or away from the retina, or bias the haptics against the surrounding tissues to reposition the lens radially within the eye, or even readjust the biasing force of the haptics to reduce unwanted bias against surrounding tissue or increase desired bias against surrounding tissue.

The stimulus with electromagnetic waves in a pre-determined designed area of the memory alloy shape induces a movement that is transferred to the proximal haptic bodies 108a and distal ends 108b, which changes the position, orientation, or intraocular lens shape, such as its curvature or vault, as desired. The nature and extent of the lens repositioning or adjusting is a function of the location of the shape memory alloy, the power of the irradiation, the time of irradiation. Lasers such as argon lasers are very useful in this regard for their ability to narrowly focus the beam of energy on a particular surface location of the alloy to bring about the desired adjustment or repositioning.

As will become apparent to one of ordinary skill in the art in view of this disclosure, numerous shape memory bars, and combinations thereof, can be used to provide for desired deformations and movements of IOL 110. More specifically, by heating different parts of the shape memory bars, a physician can, in a choreographed manner effect changes in the IOL 110 that better secure, realign, reposition or refocus an IOL in situ and without the need for surgery. Indeed, the physician can employ a laser to effect movement of IOL 110 in any number of directions, including, inter alia, radially, forward or backward, depending on the alloy, its low temperature shape and transition shape, as well as its location in the haptic.

Importantly, the transition temperature of the shape memory bars is set significantly higher than a human's normal body temperature range, and preferably higher than approximately 50 degrees Celsius. Otherwise, upon insertion into a patient, shape memory bars 111 would reach their transition temperature and cause the undesirable, uncontrolled return of the shape memory bars 111 to their original forged state. Such uncontrolled transition could displace the IOL 110 or cause other undesirable side effects.

IOL 110 is configured to be implanted in the eye as illustrated in Figures 4-6. When properly positioned, optical body 107 is centered in the pupil with the anterior lens face facing towards the posterior face of the cornea 1 and the posterior intraocular lens face facing towards the retina.

In one exemplary procedure, PIOL 110 is first inserted into the eye using standard surgical procedures. PIOL 110 (see Figure 12) is implanted in front of the "crystalline" or natural eye lens 4 and behind the iris 2 as shown in Figure 5. During the immediate post operative period, the PIOL's optical body 107 of PIOL 110 closes its distance with the natural lens 4, as shown in Figure 15 causing inadequate vaulting. After the undesired condition is identified, the patient's pupil is dilated with drops (see dilation represented in Figure 16). Some of at least one of the haptic's internal alloy shape memory devices 11 are exposed as a consequence of the dilation and are irradiated with an argon laser at specific points or segments along shape memory bars 111 with concomitant heat production. Upon reaching the transition temperature of shape memory bars 111, the bars change shape or deform. As a result, haptics 108, which contain shape memory bars 111, likewise change shape or deform. This, in turn, repositions IOL 110 to increase the distance between the natural lens and the optical body 107 of IOL 110 (Figure 17), thus creating a proper vault or space without the need to surgically remove PIOL 110.

Figure 18 shows the positioning of IOL 110 (pseudophakic) in the anterior chamber of the eye in relation to the cornea (1); central iris (2a); peripheral iris (2b); posterior chamber (3a); anterior chamber (3b); sulcus (5); ciliary zonule; and (6) in the absence of the natural lens (removed during surgery). Figure 19 similarly shows the positioning of IOL 110 (phakic) of the present invention in the anterior chamber of the eye in relation to the cornea (1); central iris (2a); peripheral iris (2b); posterior chamber (3a); anterior chamber (3b); natural lens (4); sulcus (5); and ciliary zonule (6).

Figure 20 shows an IOL 110 embodiment having multiple haptics each attached to an area of lens 107 having a proximal area 108a of haptic 108, a distal area 108b of haptic 108; and shape memory bars 111. Each memory alloy device shape is deformed in a manner to provide a specific adjustment to the lens position within at least one of three dimensions associated with the lens' position in the eye. Figure 21 shows an IOL 110 embodiment of the present invention positioned in the eye with iris dilated to expose shape memory alloy in preparation for adjustments to the IOL's positioning with laser irradiation.

In one embodiment of an IOL 110, at least one haptic 108 includes two or more shape memory bars 111. More preferably it includes two or more device memory shape alloys or at least one device memory shape alloy having multiple locations on the device to dimensionally adjust the position of the lens after the lens is implanted. Shape memory alloys are designed to alter their shape in a predetermined manner through the application of electromagnetic waves, preferably heat, preferably highly focused heat, that may be provided by use of a laser enabling a physician to specifically target where on the device heat is applied. The device preferably has multiple locations that are susceptible to the application of electromagnetic waves resulting in shape changes that affect the positioning of the lens in any of a number of directions. In this way the shape of the alloy may be manipulated to move the lens forward, back, up, down, in or out relative to the position of the natural lens or iris of the patient. The shape memory alloy is preferably designed to allow initial movements of the lens in any direction as well as revert or partially revert the lens position in situations where the lens position was initially overcorrected. This may be achieved by employing multiple hot spots on a device memory shape alloy or series of such devices where particular points in the device or devices are associated with particular movements of the lens within the implanted eye. Employing the use of highly focused electromagnetic waves, the physician can fine tune to position of the implanted lens to optimize the vision of the patient without the need for additional invasive surgery.

## Claims

1. An intraocular lens implant (110) for the correction of vision comprising:
a single flexible optical lens (107), and
one or two haptics (108) of flexible plastic material attached to the single flexible optical lens (107), wherein the one or two haptics (108) comprise at least one integral shape memory bar (111) having a shape memory alloy segment with a transition temperature between 50 degrees Celsius and 500 degrees Celsius,
**characterized in that** the integral shape memory bar (111) is provided with at least one crinkled portion (144) that coincides with an expansion zone (140) of the haptic (108), both deformable when the shape memory alloy segment reaches its transition temperature.

2. The intraocular lens implant (110) of claim 1, wherein the single flexible optical lens (107) comprises a monofocal region.

3. The intraocular lens implant (110) of claim 1, wherein the single flexible optical lens (107) comprises a multifocal region.

4. The intraocular lens implant (110) of claim 1, wherein the shape memory alloy segment is made of nickel titanium.

## Patentansprüche

1. Ein Intraokularlinsenimplantat (110) zur Sehkorrektur, umfassend:
eine einzelne flexible optische Linse (107) und
eine oder zwei Haptiken (108) aus flexiblem Kunststoffmaterial, die an der einzelnen flexiblen optischen Linse (107) angebracht sind, wobei die eine oder zwei Haptiken (108) mindestens einen integralen Formgedächtnisstab (111) mit einem Formgedächtnislegierungssegment mit einer Übergangstemperatur zwischen 50 Grad Celsius und 500 Grad Celsius umfassen,
**dadurch gekennzeichnet, dass** der integrale Formgedächtnisstab (111) mit mindestens einem gefalteten Abschnitt (144) versehen ist, der mit einer Expansionszone (140) der Haptik (108) zusammenfällt, die beide verformbar sind, wenn das Formgedächtnislegierungssegment seine Übergangstemperatur erreicht.

2. Das Intraokularlinsenimplantat (110) nach Anspruch 1, wobei die einzelne flexible optische Linse (107) einen monofokalen Bereich umfasst.

3. Das Intraokularlinsenimplantat (110) nach Anspruch 1, wobei die einzelne flexible optische Linse (107) einen multifokalen Bereich umfasst.

4. Das Intraokularlinsenimplantat (110) nach Anspruch 1, wobei das Formgedächtnislegierungssegment aus Nickel-Titan hergestellt ist.

## Revendications

1. Implant de lentille intraoculaire (110) pour la correction de la vision, comprenant :
une lentille optique flexible unique (107), et
une ou deux haptiques (108) en matière plastique flexible fixées à la lentille optique flexible unique (107), dans lesquelles la ou les haptiques (108) comprennent au moins une barre à mémoire de forme intégrale (111) ayant un segment d'alliage à mémoire de forme avec une température de transition entre 50 degrés Celsius et 500 degrés Celsius,
**caractérisé en ce que** la barre à mémoire de forme intégrale (111) est pourvue d'au moins une partie plissée (144) qui coïncide avec une zone d'expansion (140) de l'haptique (108), les deux étant déformables lorsque le segment d'alliage à mémoire de forme atteint sa température de transition.

2. Implant de lentille intraoculaire (110) selon la revendication 1, dans lequel la lentille optique flexible unique (107) comprend une région monofocale.

3. Implant de lentille intraoculaire (110) selon la revendication 1, dans lequel la lentille optique flexible unique (107) comprend une région multifocale.

4. Implant de lentille intraoculaire (110) selon la revendication 1, dans lequel le segment d'alliage à mémoire de forme est constitué de nickel-titane.
